# EUROPÄISCHE PATENTANMELDUNG

(11) **EP 0 962 523 A2**
(43) Veröffentlichungstag der Anmeldung: **08.12.1999**
(21) Anmeldenummer: 99110664.2
(22) Anmeldetag: 02.06.1999
(51) Int. Cl.: C12M 1/22, C12M 1/20

(54) **Nährbodenträger**

(30) Priorität: 03.06.1998 DE 29809928 U
(71) Anmelder: Feinchemie GmbH Sebnitz, D-01855 Sebnitz (DE)
(72) Erfinder: Kallies, Karl-Heinz, 01855-Sebnitz (DE); Löbert, Manfred, 01855-Sebnitz (DE)
(74) Vertreter: Hertz, Oliver, Dr.

(57) **Zusammenfassung**

Ein Nährbodenträger wird durch ein schalenförmiges Formteil (10 bis 90) mit einer in Standposition unten offenen Grundfläche und einer oberen Deckfläche (13) gebildet, die eine Profilierung zur Nährbodenaufnahme aufweist, wobei die Außenform des Formteils komplementär zur Innenform ist, so daß eine Mehrzahl von Nährbodenträgern dicht schließend ineinander stapelbar sind und als Trägermaterial mit dem Nährboden als Abklatschplatte herausdrückbar ist.

## Beschreibung

Die Erfindung betrifft einen Nährbodenträger und ein mehrteiliges Nährbodenträgersystem zur Züchtung, Vermehrung, Kultivierung, Lagerung oder Bestimmung von Mikroorganismen.

Es ist allgemein bekannt, daß Nährbodenträger zum Nachweis und zur Kultivierung von Mikroorganismen verwendet werden. Die allgemeinste Gestaltung von herkömmlichen Nährbodenträgern basiert auf der Form von Petrischalen, die gegebenenfalls für spezielle Anwendungen eine veränderten Schalenform aufweisen. So ist beispielsweise allgemein bekannt, daß zum Nachweis von Harnwegsinfektionen speziell zum Eintauchen entwickelte Nährbodensysteme eingesetzt werden. Diese sogenannten Eintauchkulturen bestehen aus einem Nährbodenträger, der einseitig oder doppelseitig mit einem oder mehreren verschiedenen Nährböden beschichtet ist. So ermöglicht beispielsweise eine Zweifachbe-. schickung eine Differenzierung der Mikroorganismen. Herkömmliche Nährbodenträger für Eintauchkulturen werden durch eine Versieglung der Nährbodenoberfläche mit einer Deckfolie vor dem Austrocknen geschützt. Vor der Beschickung (Eintauchen) wird die Deckfolie abgenommen.

Es sind ferner beispielsweise aus DE-AS 28 50 147, DE-OS 29 36 294, DE-GM 84 10 300 oder DE-GM 86 22 419 andere Nährbodenträger bekannt, die einen Schraubansatz aufweisen und mit einem Auffangbehälter z.B. für Urin kombiniert sind.

Weitere Nährbodenträger sind aus EP-A-111 783, EP-A-298 045, DE-GM 84 09 240, DE-GM 84 27 955, DE-OS 34 34 851 und DE-OS 43 01 882 bekannt.

Die herkömmlichen Eintauchkulturen besitzen die folgenden Nachteile. Üblicherweise dient die Trägerfläche (gegebenenfalls mit einem Schraubansatz) der manuellen Handhabung der Eintauchkultur. Dies ist nachteilig, da das Risiko einer Verunreinigung der Kultur besteht. Ferner ist die Aufbringung des Nährbodens auf die Vorder- und Rückseiten des Trägers technologisch aufwendig. Die Herstellung der herkömmlichen Nährbodenträger erfordert einen sehr präzisen Formenbau, wobei der Materialaufwand groß und damit auch der Entsorgungsaufwand erheblich ist. Zusätzliche Probleme entstehen dadurch, daß die herkömmlichen Nährbodenträger mehrteilig aufgebaut sind, was die Herstellung verkompliziert und gegebenenfalls Montageschritte erfordert. Die herkömmlichen Nährbodenträger sind nur beschränkt oder gar nicht stapelbar und weichen in der Regel bezüglich ihrer Form und Gestaltung von den Grundformen von Petrischalen stark ab. Schließlich sind die herkömmlichen Eintauchkulturen in ihrer Anwendung auf Urin und ähnliche Flüssigkeiten beschränkt.

Ein weiterer Nachteil der bisherigen Nährbodenträger ist, daß sie entweder als Eintauchkultur bzw. Ausstrichplatte oder als Abklatschplatte ausgebildet und beschichtet sind. Eine Verwendung des gleichen Nährbodenträgers sowohl als Ausstrichplatte und Abklatschplatte ist nur mit Einschränkung möglich.

Ein genereller Nachteil herkömmlicher Nährbodenträger ergibt sich aus der Bildung von Kondensatwasser während der Kultivierung oder Lagerung. Es sind bisher keine Maßnahmen zur Verhinderung von störenden Einflüssen des Kondensatwassers auf die Kulturen bekannt.

Die Aufgabe der Erfindung ist es, einen verbesserten Nährbodenträger zu schaffen, mit dem insbesondere die genannten Mängel der komplizierten technologischen Herstellung, des hohen Materialaufwandes, der aufwendigen Entsorgung und der hohen Anforderungen an die Formpräzision überwunden werden.

Diese Aufgabe wird durch einen Nährbodenträger mit den Merkmalen gemäß Anspruch 1 gelöst. Vorteilhafte Ausführungsformen der Erfindung ergeben sich aus den abhängigen Ansprüchen.

Die Erfindung basiert auf der Idee, einen Nährbodenträger (oder ein Nährbodenträgersystem, im folgenden auch: NTS) zu schaffen, der die Form eines flachen Konus oder Pyramidenstumpfes mit einer unten offenen Grundfläche, einer gegenüber der Normalen auf einer durch die Grundfläche gebildeten Ebene geneigten Wandung und einer profilierten Deckfläche derart besitzt, daß die Deckfläche auf der von der Grundfläche abgewandten Seite (Oberseite) eine Nährbodenaufnahme bildet und die Außenform des Nährbodenträgers komplementär zur Innenform des Nährbodenträgers ist, so daß eine Vielzahl von Nährbodenträgern ineinander stapelbar sind.

Der Nährbodenträger besitzt somit die Form einer flachen Schale mit rundem (Konus) oder rechteckigem (Pyramidenstumpf) Querschnitt, deren Boden die profilierte Deckfläche und gegebenenfalls weitere Bauteile wie Abdichtflächen und/oder Kondensatleitelemente umfaßt. Der erfindungsgemäße Nährbodenträger wird vorzugsweise durch ein Tiefziehverfahren hergestellt und besteht aus einem tiefziehbaren Schicht-, Folien- oder Wandmaterial, dessen Dicke verfahrens- und anwendungsabhängig ausgewählt ist und vorzugsweise kleiner als 2 mm (insbesondere 0.2 bis 1 mm) ist.

Das erfindungsgemäße NTS besitzt den Vorteil, wirtschaftlich mit geringem Materialaufwand z.B. mit dem Tiefziehverfahren aus Kunststoff herstellbar zu sein, wobei sich gleichzeitig der Entsorgungsaufwand verringert. Das Trägermaterial ist flexibel. Bei Aufliegen des Nährbodenträgers mit der Kultur über Kopf auf ein festes Substrat läßt sich jede Nährbodenfläche durch die leichte Verformbarkeit des plastischen Trägermaterials soweit vordrücken, daß ein vollständiger Kontakt jeder Nährbodenfläche zum Substrat hergestellt wird. Durch diesen Kontakt gehen alle auf dem Substrat vorhandenen Keime auf den Nährboden über. Substrate können Körper-, Arbeits-, Wand- oder Fußboden-Flächen sein. Abhängig von der Materialart und Dichte ist diese Formbarkeit regelbar, Gleichzeitig garantiert die Gestaltung des Stanzrandes und die Vertiefungen für die Nährbodenaufnahmen die notwendige Stablität des Systems an sich, so daß nur dieser Bodenteil sich beim Druck verformt und den Flächenkontakt garantiert.

Ferner sind die NTS während der Kultivierung stapelbar und durch die Dichtflächen oder -ränder der Formteile stabilisiert. Schließlich ist das erfindungsgemäße NTS effektiv gegen Austrocknung geschützt, universell anwendbar, für die Einbringung von einem oder mehreren Nährböden geeignet und nach einmaliger Benutzung leicht entsorgbar. Ein besonderer Vorteil der Erfindung ist die Stapelfähigkeit des Systems.

Weitere Einzelheiten und Vorteile der Erfindung werden im folgenden unter Bezug auf die beigefügten Zeichnungen beschrieben. Es zeigen:
- Fig. 1: Schnittansichten (oben) und Draufsichten (unten) auf Nährbodenträgersysteme gemäß einer ersten Ausführungsform der Erfindung, und
- Fig. 2: eine Schnittansicht (oben) und eine Draufsicht (unten) auf ein Zusatz- oder Deckelteil zum Verschluß eines Nährbodenträgers gemäß Fig. 1,
- Fig. 3 bis 6: Draufsichten auf Nährbodenträger gemäß weiteren Ausführungsformen der Erfindung,
- Fig. 7: Schnittansichten eines erfindungsgemäßen Nährbodenträgers zur Illustration von Elementen zur Kondensatabfuhr,
- Fig. 8: eine Schnittdarstellung zur Illustration der Kombination eines Nähbodenträgers mit einem Kulturgefäß, und
- Fig. 9: eine Schnittdarstellung einer Gruppe gestapelter, erfindungsgemäßer Nährbodenträger.

Das erfindungsgemäße Nährbodenträgersystem wird im folgenden unter Bezug auf verschiedene Ausführungsformen beschrieben. Die bei den einzelnen Ausführungsformen erläuterten Einzelheiten sind nicht auf die jeweils dargestellten Gestaltungen beschränkt, sondern auch bei den jeweils anderen Ausführungsformen implementierbar.

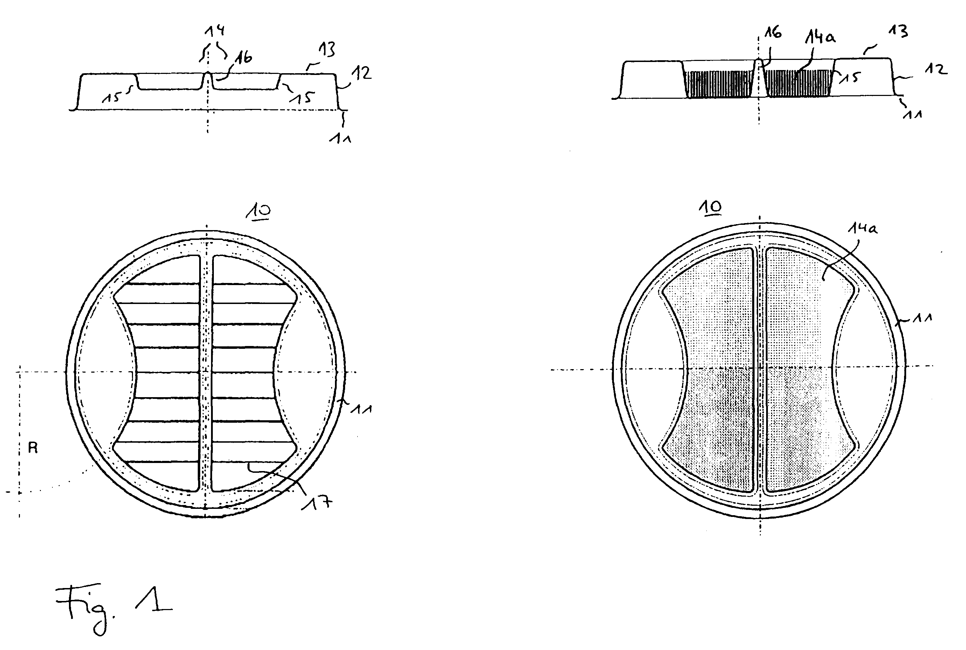

Der erfindungsgemäße Nährbodenträger gemäß einer ersten Ausführungsform besteht aus einem Formteil 10 (s. Fig. 1) in Gestalt einer profilierten Schale.

Das Formteil 10 besitzt die Gestalt eines flachen Konus mit im wesentlichen rundem Querschnitt. Der in Standposition untere Rand des Formteils (offene Grundfläche) bildet einen Auflagerand 11, der in eine nach oben hin sich verjüngende, konische Wandung 12 übergeht. Der Auflagerand 11 und/oder die Wandung 12 bilden eine Auflage für die Innenseite eines weiteren, gleichartig gestalteten Nährbodenträgers (Stapelung) oder des fakultativ einsetzbaren Deck- oder Zusatzteils 20 (s. unten). Nach oben hin besitzt das Formteil 10 eine Deckfläche 13 mit einer Profilierung, die auf der Oberseite der Deckfläche 13 mindestens zwei Ausnehmungen 14 für Nährbodentlächen und auf der Unterseite der Deckfläche 13 mindestens zwei Vorsprünge 15 als Griffelemente bildet. Die Ränder des Nährbodenträgers und des Deckels sind gleich breit oder unterschiedlich breit ausgebildet. Vorteilhaft für die Stapelung und Handhabung ist eine unterschiedliche Breite der Ränder.

Die Tiefe der Ausnehmung bzw. Höhe der Vorsprünge ist entweder geringer als die Höhe der äußeren, umlaufenden Wandung 12 und entspricht anwendungsabhängig der jeweils gewünschten Nährbodenschichtdicke (linker Teil von Fig. 1). Sie ist vorzugsweise 1 bis 3 mm niedriger ausgeführt als die Höhe der äußeren Wandung und ermöglicht so die Stapelfähigkeit. Im rechten Teil von Fig. 1 ist zusätzlich in den Ausnehmungen 14 der Nährboden 14a dargestellt. Ist die Nährbodenschichtdicke beispielsweise rd. 4 bis 5 mm, so sollten auch die Ausnehmung oder Vertiefung für die Nährbodentläche rd. 4 bis 5 mm tief oder sogar noch geringfügig (bis zu rd. 3 bis 4 mm) tiefer sein.

Der Steg 16 zwischen den Ausnehmungen 14 für die Nährbodenflächen dient der Unterteilung des Nährbodenträgers in mehrere Fächer. Im dargestellten Fall sind es zwei Fächer, die entsprechend mit zwei verschiedenen Nährböden befüllt werden können. Alternativ ist aber auch die Anbringung von mehr Stegen zur Unterteilung in mehr als zwei Fächer möglich.

Auf dem Boden jeder Ausnehmung 14 ist ein Steg- oder Strich-Raster 17 vorgesehen. Das Raster 17 besitzt eine Doppelfunktion. Erstens verbessert es die Haftung des Nährbodens zum Nährbodenträger. Zweitens ergibt sich eine Unterteilung der Fächer, die eine Keimzählung vereinfacht. Das Raster 17 ist vorzugsweise so gebildet, daß die Unterteilung der Fächer in Teilflächen von jeweils rd. 1 cm² ergibt.

An den vom Steg 16 wegweisenden Seiten der Deckflächenprofilierung bilden die Vorsprünge 15 Griffelemente zur Handhabung z.B. mit zwei Fingern. Zum sicheren Griff ist die Profilierung mit einer zum Steg 16 hinweisenden Krümmung der Profilierungsstufe (s, unterer Teil von Fig. 1) versehen.

Bei einer praktisch realisierten Ausführungsform wird das Unterteil beispielsweise mit den folgenden Maßen hergestellt. Außenradius des Auflagerandes 11: rd. 35 bis 50 mm, Außenradius der konischen Wandung 12: rd. 30 bis 40 mm, Höhe der Wandung 12: 5 bis 12 mm, Breite des Stegs 16: rd. 2 bis 8 mm, Radius der Griffelemente 15a: rd. 15 bis 20 mm, Gesamtnährbodenfläche rd. 10 bis 20 cm² oder ein Vielfaches von 10 cm².

Die gegenüber der Normalen auf der durch die Grundfläche bzw. den Rand 11 gebildeten Bezugsebene geneigten Seitenwände des Formteils 10 bilden Dichtflächen beim Stapeln von Nährbodenträgern in Form eines Nährbodenträgersystems. Die Neigung beträgt beispielsweise rd. 1 bis 4°. Beim stapelförmigen Ineinandersetzen oder -schieben von Nährbodenträger-Formteilen berührt jeweils die geneigte Außenwand des unteren Formteils die geneigte Innenwand des oberen Formteils. Je nach anwendungsabhängig gewählter Dicke und Neigung der Seitenflächen lassen auch fest ineinander geschobene oder gestapelte Nährbodenträger jeweils einen Innenraum frei, der eine Probenkultivierung auch im Stapel erlaubt. Dies ermöglicht eine platzsparende Probenkultivierung für eine Vielzahl verschiedener Proben in einem Nährbodenträgerstapel.

Es kann vorgesehen sein, einen einzelnen Träger oder einen Trägerstapel nicht mit einem unbeschickten Nährbodenträger, sondern mit einem Deck- oder Zusatzteil 20 an oberster Stelle zu verschließen. Das Deckteil 20 ist jedoch nicht zwingend erforderlich.

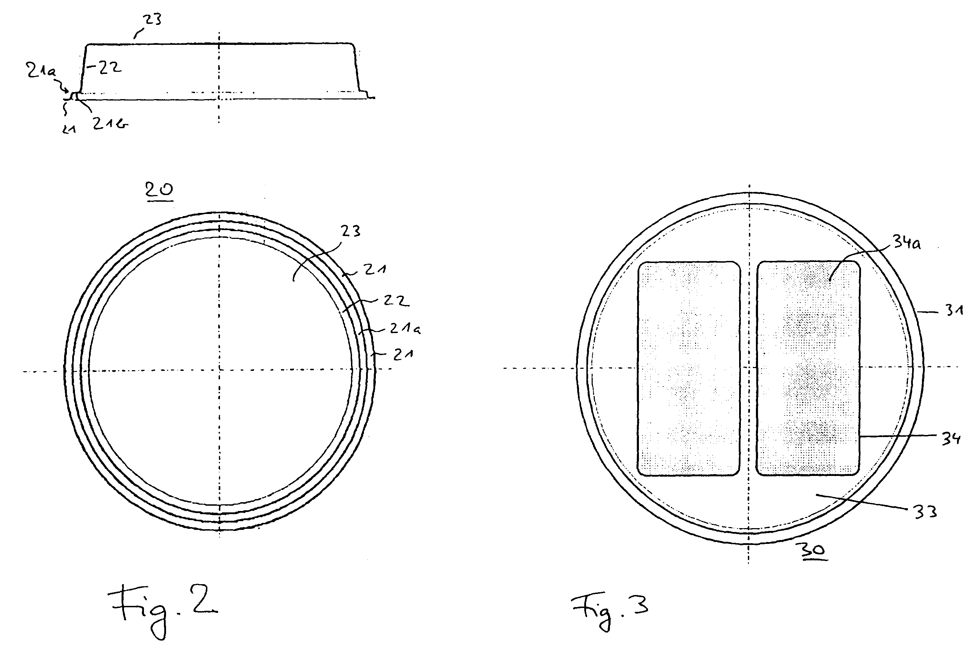

Das Deckteil 20 ist gemäß Fig. 2 als zum Formteil 10 passender Deckel ausgeführt, dessen Höhe so gewählt ist, daß ein genügend großer Raum für ein ungestörtes Wachstum der Mikroorganismen auf den Nährbodenflächen gegeben ist. Dieser Raum sollte rd. 3 bis 5 mm betragen. Das Oberteil 20 besitzt ebenfalls einen Auflagerand 21, der über eine Verschlußauflage 21a in die Wandung 22 übergeht. Die Wandung 22 ist wiederum konisch mit sich in Standposition nach oben hin verjüngendem Durchmesser entsprechend der Wandung 12 des Unterteils 10 ausgebildet.

Die Deckfläche 23 ist eben und kann als Auflage für die Unterseite eines Formteils 10 eines weiteren Nährbodenträgersystems bei stapelweiser Lagerung dienen. Die Verschlußauflage 21a bildet eine umlaufende Stufe. Die entsprechende umlaufende Vorsprungsfläche 21b ist als Verbindungsbereich mit dem Auflagerand 11 des Unterteils vorgesehen. Hierzu ist der Auflagerand 11 des Unterteils und/oder die umlaufende Vorsprungsfläche 21b mit einem geeigneten Klebemittel versehen, das beim Zusammensetzen der Form- und Deckteile 10, 20 einen sicheren Verschluß liefert, so daß ein abgeschlossener Brutraum für das ungestörte Wachstum der Kolonien auf den Nährbodenflächen gebildet wird. Entsprechende Vorsprungsflächen können auch an den Formteilen 10 selbst vorgesehen sein, um einen entsprechenden Verschluß nicht nur zwischen den Form- und Deckteilen, sondern auch zwischen den Formteilen untereinander zu ermöglichen.

Die Maße des Deckteils 20 sind an die Maße des Unterteils 10 (s. oben) angepaßt. Die Verschlußauflage 21a besitzt gegenüber dem Auflagerand 21 eine Höhe von rd. 1 bis 3 mm und eine Tiefe von rd. 1 bis 3 mm.

Die Teile des erfindungsgemäßen NTS werden vorzugsweise durch ein Tiefziehverfahren hergestellt und bestehen z.B. aus Kunststoff. Der Kunststoff kann durchsichtig oder gefärbt sein, wobei im Fall des Deckteils 20 ein durchsichtiger Kunststoff zur Sichtprüfung der Kolonien bevorzugt wird. Es wird vorzugsweise ein Kunststoff verwendet, der strahlen- oder dampfsterilisierbar ist, ohne sich zu verfärben oder zu verformen. Der Kunststoff kann anwendungsabhängig eine relativ hohe Härte oder Steifigkeit oder eine gewisse Elastizität und Verformbarkeit besitzen. Gemäß einer bevorzugten Ausführungsform der Erfindung besteht das Formteil aus plastisch tiefziehbarem Material, das mit einer geeigneten Beschickungseinrichtung (z.B. Injektionsnadel oder Injektionskapillare) durchstoßen werden kann und sich nach Entfernen der Beschickungseinrichtung selbständig elastisch verschließt.

Die Benutzung des NTS erfolgt derart, daß zunächst die Ausnehmungen 14 mit einem Nährboden beschickt werden. Anschließend erfolgt die Impfung der Nährbodentläche durch Übergießen oder Ausstrich, Daraufhin werden die Formteile 10 untereinander oder Deckteile 10, 20 zusammengesetzt und der Auflagerand 11 des Formteils 10 gegen die umlaufende Vorsprungsfläche 21b des Deckteils 20 gedrückt. Das Klebemittel sichert einen Verschluß des Innenraumes. Ersatzweise kann ein Verschweißen der aneinanderliegenden Flächen vorgesehen sein. Eine Vielzahl von NTS kann nach Beschickung und Beimpfung und Verschluß unter geeigneten Lagerbedingungen gestapelt werden. Auf der Deckfläche 23 des Deckteils 20 kann zusätzlich eine Anzeigeeinrichtung zur Beschriftung vorgesehen sein.

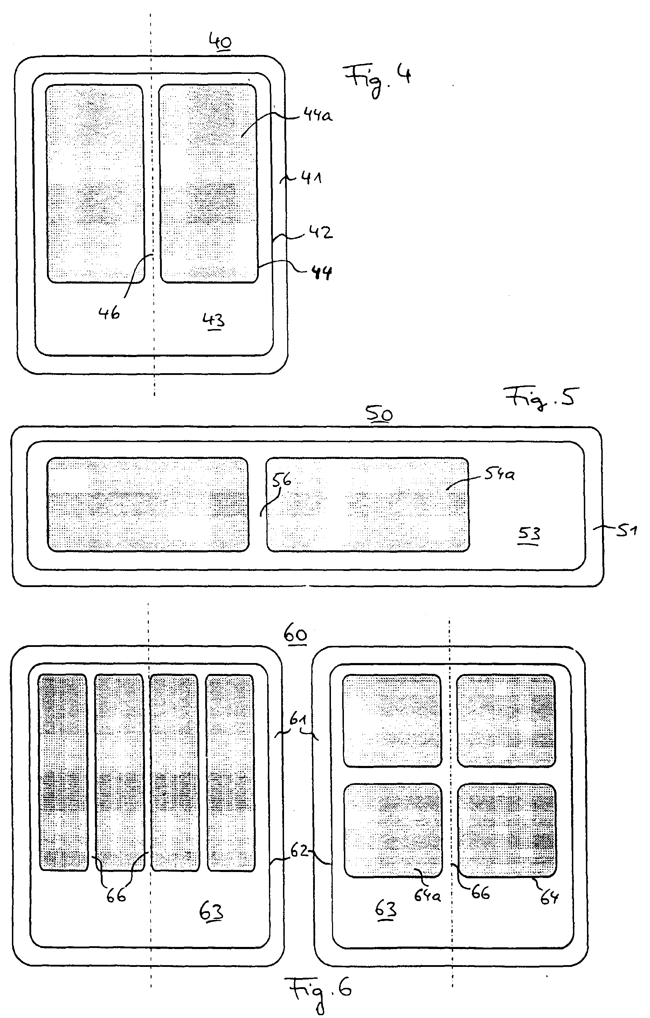

Fig. 3 zeigt einen erfindungsgemäßen Nährbodenträger in einer abgewandelten Ausführungsform. Der Nährbodenträger besteht wiederum aus einem Formteil 30, das im wesentlichen konus- oder schalenförmig ist und einen kreisrunden Querschnitt besitzt. Im Unterschied zum Formteil 10 gemäß Fig. 1 sind bei der Gestaltung gemäß Fig. 3 Ausnehmungen 34 mit rechteckiger Grundfläche vorgesehen. In den Ausnehmungen 34 ist wiederum der Nährboden 34a (gepunktet dargestellt) vorgesehen. Die übrigen Einzelheiten des Nährbodenträgers können so wie beim Formteil 10 ausgebildet sein.

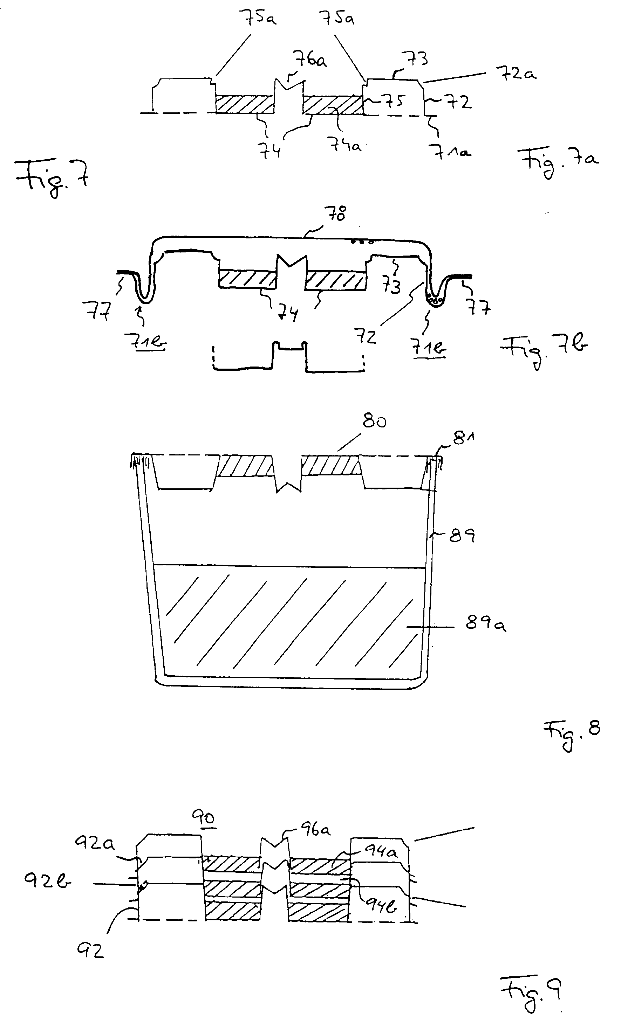

Es ist erfindungsgemäß nicht zwingend erforderlich, daß der Nährbodenträger einen kreisrunden Querschnitt besitzt. Vielmehr können auch andere, kurvenförmige oder gerade Querschnittsformen implementiert werden. Beispiele für quadratische oder rechteckige Nährbodenträger sind in den Fig. 4 bis 6 in schematischer Draufsicht dargestellt. Die Nährbodenträger bestehen jeweils aus Formteilen 40, 50, 60 mit einer rechtekkigen Grundform. Es ist jeweils ein Auflagerand 41, 51, 61 vorgesehen, der eine Dichtfläche bilden kann. An einer Seite des Formteils kann der jeweilige Rand breiter ausgebildet sein als an den übrigen Seiten. Damit wird seitlich eine Zusatzfläche geschaffen, die als Griff zum Anfassen und Aufdeckeln des Nährbodenträgers und/oder als Beschriftungsfläche verwendet werden kann. Die Beschriftung kann durch direktes Bedrucken des an der Seite erweiterten Randes oder durch Aufkleben eines beschrifteten Schildes erfolgen. Vom jeweiligen Rand geht das Formteil über die Seitenflächen 42, 52 bzw. 62 zu den oberen Deckflächen 43, 53 bzw. 63 über, in denen jeweils die Ausnehmungen 44, 54 bzw. 64 vorgesehen sind. Die Ausnehmungen besitzen verschiedene Grundflächen. Es können anwendungsabhängig verschieden geformte Ausnehmungen mit verschiedenen Gesamtzahlen vorgesehen sein, die jeweils durch Stege 46, 56, 66 getrennt sind. Auch die rechteckigen Nährbodenträger gemäß den Fig. 4 bis 6 sind nach den oben erläuterten Prinzipien stapel- und verschließbar. Es kann auch ein rechteckiges Deckteil analog zu dem Deckteil gemäß Fig. 2 vorgesehen sein.

Die oberen Deckflächen 43, 53 bzw. 63 können abweichend von der dargestellten Form ohne eine Strukturierung alternativ auch Profile in Form von Noppen oder Stegen aufweisen, die über die jeweiligen oberen Deckflächen hinausragen. Diese Profile sind dazu ausgebildet, im gestapelten Zustand über dem Nährbodenträger einen Freiraum zu bilden, durch den der Gasaustausch der Kultur gefördert wird. Es sind beispielsweise an den Ecken der oberen Deckflächen vier hervorragende Noppen vorgesehen, auf denen im gestapelten Zustand der jeweils nächste Nährbodenträger aufliegt. Das Profil der oberen Deckfläche wird simultan zur Profilierung des übrigen Nährbodenträgers beim Tiefziehvorgang ausgebildet.

Fig. 7 dient der Illustration eines wichtigen Merkmals der Erfindung, nämlich der Bereitstellung von Elementen zur Kondensatabführung. Fig. 7a zeigt eine schematische Schnittansicht eines Nährbodenträgers beispielsweise gemäß Fig. 1 mit dem Rand 71a, der Seitenwand 72 und der oberen Deckfläche 73, deren Profilierung die Ausnehmungen 74 jeweils mit den Nährböden 74a bildet. Zusätzlich zu den Darstellungen gemäß Fig. 1 ist an der Oberseite des Steges 76 eine Kondensatrinne oder Auffangrinne 76a erkennbar. Ferner ist am oberen Ende der Seitenwand 72 ein Kondensatspalt 72a vorgesehen. Diese Elemente zum Schutz der Kulturen vor Kondensat dienen der Kondensataufnahme. In der Kondensatrinne 76a bzw. im Kondensatspalt 72a können sich Kondensattröpfchen (s. Fig. 9) ansammeln. Durch die erfindungsgemäße Profilierung der Deckfläche bzw. der Stege werden die Kondensattröpfchen gehindert, zu den Kulturen zu gelangen.

In Fig. 7a ist außerdem am oberen Rand der Wand 75 der Ausnehmungen 74 eine umlaufende Folienauflage 75a vorgesehen, die zur Anbringung einer Klebefolie zum Verschluß der Auflagen 74 eingerichtet sind. Es ist vorzugsweise eine einzelne Klebefolie vorgesehen, die alle Ausnehmungen 74 und die Trennstege 76 überdeckt. Die Anbringung einer Klebefolie ist insbesondere dann vorgesehen, wenn die Nährbodenträger nicht gestapelt, sondern einzeln gelagert werden.

Weitere Elemente zur Kondensatabführung sind in Fig. 7b anhand einer schematischen Schnittansicht eines modifizierten Nährbodenträgers dargestellt. Die Modifizierung betrifft eine abgewandelte Gestalt des Randes 71b. Die übrigen Komponenten, insbesondere die Seitenwand 72, die obere Deckfläche 73 und die Ausnehmungen 74 sind im wesentlichen wie bei den oben beschriebenen Ausführungsformen ausgebildet. Der Rand 71b ist nicht als flacher, umlaufender Streifen, sondern selbst als Rinne ausgebildet. Die Seitenwand 72 geht über diese Rinne zu einem äußeren Stanzrand 77 über, der die Gestalt und Funktion des Randes 71 der obengenannten Ausführungsformen übernimmt. Diese Ausführungsform eines erfindungsgemäßen Nährbodenträgers ist zur Kombination mit einem Deckel 78 vorgesehen, dessen äußere Umrandung an den rinnenförmigen Rand 71b angepaßt ist. Der Deckel 78 ist so dimensioniert, daß im aufgesetzten Zustand zwischen dem Boden des Randes 71 und dem in die Rinne hereinragenden Deckel 78 ein Spiel von rd. 1 mm verbleibt. Der Rand 71 besitzt die Funktion einer Überlaufrinne. Kondensattröpfchen an der Innenseite des Deckels 78 laufen in diese Überlaufrinne und werden dort gesammelt. Damit werden die Kondensattröpfchen von der Kultur ferngehalten, Simultan wird vorteilhafterweise eine Abdichtung des abgedeckelten Nährbodenträgers gegenüber der Umgebungsluft erzielt.

Gemäß einer weiteren Ausführungsform der Erfindung kann die Auffangrinne 76a (s. Fig. 7a) als Einlagefach (siehe unterer Ausschnitt in Fig. 7b) verbreitert zur Aufnahme eines Indikatorstreifens oder einer Reaktionshilfe ausgeführt sein. Die Reaktionshilfe kann beispielsweise ein Oxidasestreifen oder dergleichen sein, der bei Bedarf aus der Rinne 76a entnommen und auf die Kultur aufgelegt wird.

Fig. 8 illustriert die Kombination eines erfindungsgemäßen Nährbodenträgers (beispielsweise gemäß Fig. 1 oder Fig. 7) mit einem Vorrats- oder Kulturgefäß, das eine flüssige Nährlösung 89a enthält. Das Formteil 80 ist wie die Formteile bei den anderen Ausführungsformen entsprechend gestaltet, wobei die Grundform des Nährbodenträgers, der mit dem Rand 81 auf dem oberen Rand des Vorratsgefäßes 89 aufliegt, an die Gestalt des Vorratsgefäßes angepaßt ist. Zwischen dem Rand 81 und dem Vorratsgefäß 89 kann eine Klebe- oder Schweißverbindung vorgesehen sein. Bei der Gestaltung gemäß Fig. 8 bildet der erfindungsgemäße Nährbodenträger eine nährbodenbeschichtete Deckplatte für das Vorratsgefäß.

Fig. 9 zeigt als Beispiel für ein Nährbodenträgersystem drei übereinander gestapelte Nährbodenträger, deren Einzelheiten jeweils entsprechend den oben erläuterten Ausführungsformen ausgebildet sind. Der Kondensatspalt 92a ist jeweils zur Sammlung von Kondensattröpfchen 92b zwischen der Außenwand des unteren und der Innenwand des oberen Nährbodenträgers eingerichtet, Weiteres Kondensat kann sich in den Kondensatrinnen 96a sammeln. Im übereinander gestapelten Zustand bilden die Formteile 90 jeweils Dichtkontakte 92 zwischen der Außenwand des unteren und der Innenwand des oberen Formteils. Zwischen dem Nährboden 94a und der Bodenfläche der Ausnehmung 94 des nächstfolgenden Nährbodenträgers entsteht jeweils ein Hohlraum 94b mit einer lichten Höhe von vorzugsweise 4 bis 5 mm. Die Kondensatrinne 96a kann beispielsweise 0.5 bis 2 mm breit und 0.5 bis 3 mm tief sein. Für die Kondensatspalte werden als Dimensionen Größen im Bereich 0.5 bis 3 mm jeweils für die Höhe oder Breite bevorzugt.

Mit dem erfindungsgemäßen Nährbodenträger werden folgende Vorteile erzielt. Der Nährbodenträger zeichnet sich durch ein materialsparendes Verfahren aus. Der Nährbodenträger ist universell als Tauch-, Abklatsch- und Impf-Platte gleicherweise gut geeignet. Das Abklatschen erfolgt aus dem vertieft eingegossenen Nährboden durch Druck auf den dünnen Plastikboden und Andruck an das Substrat. Es ist kein Nährbodenüberstand erforderlich. Die Nährbodenplatten sind raumsparend stapelbar ausgebildet. Das führt zur besseren Ausnutzung der Brutschrankkapazität. Es führt außerdem zu einer raumsparenden Verpackung und vermindert den Transportaufwand. Auch der Entsorgungsaufwand reduziert sich erheblich. Die Kondenswasserbildung wird stark verringert, so daß der Bewuchs auf den Platten abgescannt und die Ergebnisse archiviert werden können. Der Arbeitsablauf ist in rationeller und vorteilhafter Weise von der Folienrolle zum fertigkonfektionierten Nährboden als automatisierte Verarbeitsstrecke ausführbar.

## Patentansprüche

1. Nährbodenträger, der durch ein schalenförmiges Formteil (10 bis 90) mit einer in Standposition unten offenen Grundfläche und einer oberen Deckfläche (13) gebildet wird, die eine Profilierung zur Nährbodenaufnahme aufweist, wobei die Außenform des Formteils komplementär zur Innenform ist, so daß eine Mehrzahl von Nährbodenträgern dicht schließend ineinander stapelbar sind.

2. Nährbodenträger gemäß Anspruch 1, bei dem das Formteil (10 bis 90) die Form eines flachen Konus oder Pyramidenstumpfes mit einer Wandung (12, 22) besitzt, die gegenüber der Normalen auf der Grundfläche geneigt ist.

3. Nährbodenträger gemäß Anspruch 1 oder 2, bei dem die Profilierung der Deckfläche eine Mehrzahl von Aufnahmen (14) bildet, die durch Stege (16) getrennt sind.

4. Nährbodenträger gemäß Anspruch 3, bei dem die Stege (16) und/oder der obere Rand der Wandung (12) des Formteils Kondensatleitelemente aufweisen.

5. Nährbodenträger gemäß einem der vorhergehenden Ansprüche, bei dem das Formteil ein Tiefziehformteil ist.

6. Nährbodenträger gemäß einem der vorhergehenden Ansprüche, der aus einer geformten Folie mit einer Dicke von 0,2 bis 0,5 mm besteht.

7. Nährbodenträger gemäß einem der vorhergehenden Ansprüche, bei dem die Ausnehmungen (14) mit einer Rasterstruktur (17) versehen sind.

8. Nährbodenträger gemäß einem der vorhergehenden Ansprüche, der dazu ausgebildet ist, daß der vertieft vorliegende Nährboden mit dem Boden des Nährbodenträgers an eine Substratfläche als Abklatschmedium andrückbar ist.

9. Nährbodenträger gemäß einem der vorhergehenden Ansprüche, bei dem das Formteil aus durchsichtigem oder gefärbtem Kunststoff besteht.

10. Nährbodenträger gemäß einem der vorhergehenden Ansprüche, bei dem die Wandung (12) des Formteils (10) an der Grundfläche in einen außen umlaufenden Rand (11) übergeht.

11. Nährbodenträger gemäß Anspruch 10, bei dem der umlaufende Rand ein rinnenförmiger Rand (71b) ist, der ein Kondensatsammelelement bildet.

12. Nährbodenträger gemäß einem der vorhergehenden Ansprüche, bei dem an der Profilierung zur Nährbodenaufnahme ein verbreiterter Steg (76a) zur Aufnahme von Indikatorstreifen oder Reaktionshilfen vorgesehen ist.

13. Nährbodenträgersystem, bestehend aus einer Vielzahl von ineinander gestapelten Nährbodenträgern gemäß einem der Ansprüche 1 bis 12.
